# EUROPÄISCHE PATENTSCHRIFT

(11) **EP 1 759 654 B1**
(45) Veröffentlichungstag und Bekanntmachung des Hinweises auf die Patenterteilung: **23.04.2008**
(21) Anmeldenummer: 05019271.5
(22) Anmeldetag: 05.09.2005
(51) Int. Cl.: A61C 3/02, A61C 8/00

(54) **Dentalbohrvorrichtung mit einem Stoppelement**
Dental drilling apparatus with stopping element
Dispositif dentaire d'alésage avec butée

(43) Veröffentlichungstag der Anmeldung: 07.03.2007
(60) Teilanmeldung: 07017526.0
(73) Patentinhaber: Straumann Holding AG, 4002 Basel (CH)
(72) Erfinder: Müller, Daniele, 4102 Binningen (CH); Kenk, Frank, 79576 Weil am Rhein (DE)
(74) Vertreter: Schaad, Balass, Menzl & Partner AG

(56) Entgegenhaltungen:
- EP-A- 0 896 812
- US-A- 5 429 504
- US-A1- 2004 092 940
- US-B1- 6 739 872

## Beschreibung

Die vorliegende Erfindung betrifft eine Dentalbohrvorrichtung mit einem Bohrer und einem Stoppelement.

Eine solche Vorrichtung ist insbesondere in der US 2004/0092940 offenbart.

Eine weitere Dentalbohrvorrichtung dieser Art ist aus US 5,890,897 bekannt. Diese Druckschrift offenbart eine Dentalbohrvorrichtung mit einem an einen Bohrer gehaltenen Stoppelement. Das Stoppelement umgreift den Bohrer in Umfangsrichtung vollständig und ist in Axialrichtung verschiebbar an diesem gehalten.

Der Bohrer weist ein an einem Schneidteil freiliegendes Bohrende und einen Zylinderschaft mit einer freiliegenden schaftstirnfläche auf. Zwischen dem Bohrende und der Schaftstirnfläche befindet sich ein Halteabschnitt mit mehreren in Axialrichtung von einander beabstandeten und zueinander ausgerichteten, kugelhaubenartigen Befestigungsvertiefungen.

Das Stoppelement weist eine kreiszylindrische Form mit einer Bohranschlagsfläche auf. In Axialrichtung ist das Stoppelement mit einer zentralen Durchgangsöffnung ausgeführt, welche im Wesentlichen einem Durchmesser des Bohrers entspricht. In radialer Richtung des Stoppelementes führt eine Montageöffnung durch das Stoppelement. In der Montageöffnung ist ein federbelastetes, stiftartiges Eingriffselement angeordnet.

Das Stoppelement ist von dem Bohrende her auf den Bohrer aufsetzbar und an diesem durch Eingriff des Eingriffselementes in eine der Befestigungsvertiefungen kraftschlüssig fixierbar.

Die Bohranschlagfläche ist folglich in einem definierten, gewünschten Abstand zum Bohrende angeordnet, wodurch eine maximale Bohrtiefe festgelegt ist. Beim Bohren eines Lochs in einen Kieferknochen steht die Bohranschlagfläche beim Erreichen der gewünschten Bohrtiefe an den Kieferknochen bzw. an das Zahnfleisch an.

Weiter offenbart die US 5, 890, 897 eine alternative Ausführungsform des Stoppelements. Bei dieser Ausführungsform ist das Eingriffselement mit einem Aussengewinde versehen und in die Montageöffnung eingeschraubt. Durch Ausrichtung des Eingriffselementes mit einer der Befestigungsyertiefungen und anschliessendem Drehen des Eingriffselements gelangt dieses in formschlüssigen Eingriff mit der betreffenden Befestigungsvertiefung.

In EP 0 643 567 B1 ist ein Bohrer offenbart, welcher in Axialrichtung voneinander beabstandete, in Umfangsrichtung verlaufende, ringförmige Markierungen aufweist, die, sich farblich von der Schicht der Oberfläche des Bohrers unterscheiden. Die ringförmigen Markierungen dienen beim Bohren zum Abschätzen der aktuellen Bohrtiefe. Die Ringe sind durch eine Oberflächenbehandlung des Bohrers hergestellt.

Die US 5,941,706 offenbart einen Bohrer, welcher in Umfangsrichtung verlaufende, in Axialrichtung voneinander beabstandete, farbige Ringe aufweist. Hierzu weist der Bohrer in Axialrichtung voneinander beabstandete ringförmige Nuten auf, welche mit eingefärbtem Material gefüllt sind. Beim Bohren ist mit Hilfe dieser farbigen Ringe die aktuelle Bohrtiefe ablesbar.

Die US 6,514,258 B1 offenbart eine Dentalbohrvorrichtung umfassend einen Bohrer und ein Stoppelement. Der Bohrer weist ein Bohrende und einen Zylinderschaft auf. An einem dem Bohrende zugewandte Endbereich des Zylinderschafts, weist dieser eine manschettenartige Verdickung auf. Diese Verdickung ist in Richtung des Bohrendes durch eine sich verjüngende Kegelstumpffläche begrenzt. Das Stoppelement ist hülsenförmig ausgebildet und weist an einer ihrer Stirnseiten eine Bohranschlagsfläche und angrenzend an eine andere Stirnseite einen Klemmbereich auf, der dazu bestimmt ist, die Verdickung zu umgreifen. Das Stoppelement ist von dem Bohrende her auf den Bohrer aufschiebbar, wodurch der Klemmbereich schnappverschlussartig auf die Verdickung aufgesteckt wird. Die Stoppelemente sind entsprechend unterschiedlicher Bohrtiefen unterschiedlich lang ausgeführt.

Die US 6,739,872 B1 offenbart eine Dentalbohrvorrichtung bestehend aus einem Bohrer und einem den Bohrer hülsenartig umgreifenden Stoppelement. Der Bohrer weist einen Schneidteil mit einem freiliegenden Bohrende und einen Zylinderschaft auf. Zwischen dem Schneidteil und dem Zylinderschaft ist ein Halteabschnitt ausgebildet. Der Halteabschnitt weist ein Gewinde auf, welches gegenläufig zur Windung der Spannuten des Spiralbohrers ausgebildet ist. Das Stoppelement ist vom Bohrende her mit einem Innengewinde voraus auf den Bohrer aufgesteckt und auf das Gewinde aufgeschraubt. Dem Bohrende zugewandt weist das Stoppelement eine freiliegende Bohranschlagsfläche auf. Um eine maximale Bohrtiefe festzulegen, wird das Stoppelement durch Drehen an die gewünschte axiale Position verbracht. Sobald beim Bohren die Bohranschlagsfläche durch Zusammenwirken mit einem Material, in welches sich der Bohrer einbohrt, gestoppt beziehungsweise am Mitdrehen mit dem Bohrer gehindert wird, wird infolge des Gewindes der Bohrer automatisch aus dem Bohrloch herausbewegt.

Die bekannten Dentalbohrvorrichtungen bzw. Dentalbohrer weisen unterschiedliche Nachteile auf. Diejenigen Bohrer, welche nur mit optischen Elementen zur Bestimmung der Bohrtiefe ausgeführt sind, haben den Nachteil, dass beim Gebrauch die aktuelle Bohrtiefe nur ungenau feststellbar ist und die optische Markierung unlesbar verschmutzt sein kann.

Bei Dentalbohrvorrichtungen mit einem Stoppelement kann sich die Handhabung kompliziert gestalten, wie beispielsweise bei jener gemäss US 6,514,258 B1. Weil die Länge des Bohrstoppelementes an die jeweilige Bohrtiefe des zu bohrenden Lochs individuell angepasst werden muss und eine Wiederverwendung eines benutzten Bohrstoppelementes nicht möglich ist.

Bei der in US 5,890,897 offenbarten Dentalbohrvorrichtung kann im Einsatz, wenn das Stoppelement mit der Bohranschlagfläche am Kieferknochen bzw. am Zahnfleisch ansteht, der Kraftschluss überwunden werden, was zu einer zu tiefen Bohrung führen würde.

Aufgabe der vorliegenden Erfindung ist es, eine Dentalbohrvorrichtung vorzuschlagen, bei welcher die Lage der Bohranschlagfläche bezüglich des Bohrers und somit die Tiefe des zu bohrenden Lochs zuverlässig und in einfacher Weise festlegbar ist.

Weiter sollen sämtliche Elemente der erfindungsgemässen Dentalbohrvorrichtung wiederverwendbar sein.

Diese Aufgaben werden erfindungsgemäss mit einer Dentalbohrvorrichtung gemäss Anspruch 1 gelöst.

Durch die zumindest annähernd rechtwinklig zur Achse des Bohrers ausgerichtete Anschlagsfläche und durch das Eingriffselement, welches eine mit der Anschlagsfläche formschlüssig zusammenwirkende Gegenanschlagfläche aufweist, ist sichergestellt, dass das Stoppelement, bei Krafteinwirkung in Axialrichtung und weg vom Bohrende auf das Stoppelement, unbeweglich am Verlängerungselement gehalten ist.

In einer bevorzugten Ausführungsform weist das Stoppelement eine radial aussenliegende kreiszylindrische Führungsoberfläche auf. Dadurch ist es ermöglicht, das Stoppelement und somit den Bohrer beispielsweise an einer Bohrlehre zu führen, wodurch die Lage und Richtung der zu erzeugenden Bohrung genau vorgegeben sein kann.

In einer weiteren bevorzugten Ausführungsform weist die Dentalbohrvorrichtung eine Bohrlehre auf, welche bezüglich einer Mundhöhle eines Patienten ortsfest fixierbar ist. Die Bohrlehre weist eine kreiszylindrische Ausnehmung mit einer Gegenführungsfläche auf, welche dazu bestimmt ist mit der Führungsoberfläche ztasarnmenzuwirken. Durch dieses Zusammenwirken ist die Bohrrichtung und Lage der Bohrung durch die Bohrlehre gegeben. Eine Fehlrichtung und Fehllage der Bohrung wird verhindert.

In einer weiteren bevorzugten Ausführungsform weist die Bohrlehre weiter eine gegenüber der Gegenführungsfläche radial nach innen vorstehende Schulter auf, die dazu bestimmt ist mit der Bohranschlagsfläche des Stoppelements zusammen zu wirken. Durch diese Ausführungsform ist eine maximale Bohrtiefe durch Zusammenwirken der Schulter mit der Bohranschlagsfläche festgelegt.

In einer weiteren bevorzugten Ausführungsform ist das Stoppelement hülsenartig ausgebildet und das Eingriffselement ist vorzugsweise an einem als Federfinger ausgebildeten Federelement angeordnet. Durch diese Ausführungsform ist eine in radialer Richtung kompakte Bauform ermöglicht. Weiter ist durch diese Ausführungsform ein Aufstecken der Stoppelements vom Aufnahmeendbereich her ermöglicht, da ein auf den Halteabschnitt aufgestecktes Stoppelement mit dem in eine der Aufnahmevertiefungen eingreifenden Eingriffselement durch Überwinden einer bestimmten Kraft in Richtung des Bohrendes verschiebbar ist und dadurch das Eingriffselement in eine andere der Aufnahmevertiefungen verbringbar ist.

Weitere bevorzugte Ausbildungsformen der erfindungsgemässen Dentalbohrvorrichtung sind in den weiteren abhängigen Ansprüchen angegeben.

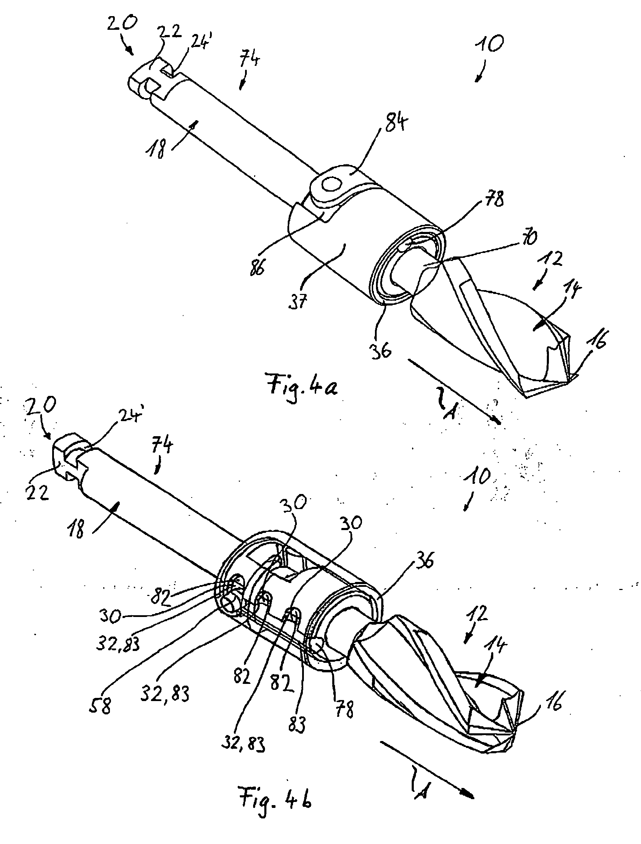

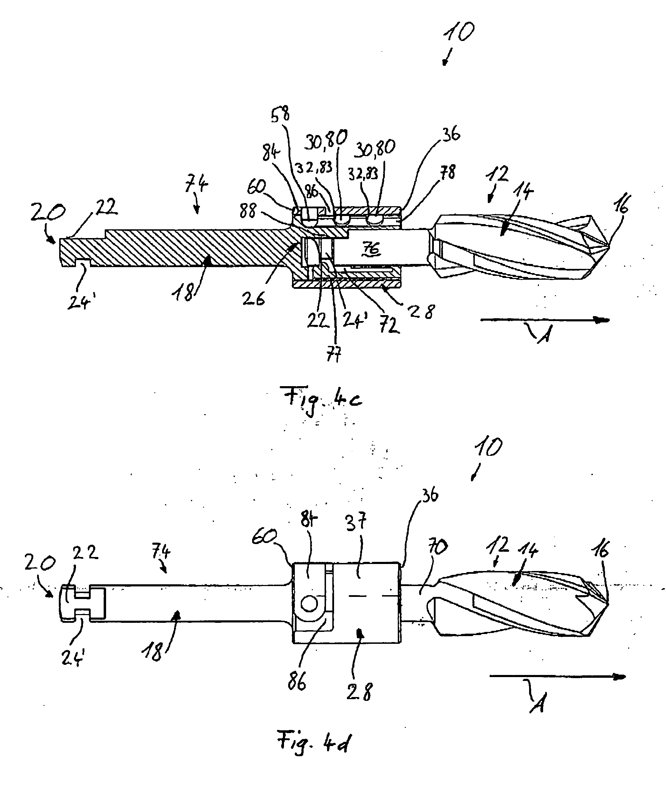

Weitere besondere Vorteile und Wirkungsweisen ergeben sich aus der Detailbeschreibung und der Zeichnung, wobei lediglich die Figuren 4a, 4b, 4c und 4d erfindungsgemäße Ausführungsformen darstellen.

Im Folgenden wird die Erfindung anhand in der Zeichnung dargestellten Ausführungsbeispielen näher erläutert. Es zeigen rein schematisch:
- Fig. 1: In perspektivischer Darstellung eine Dentalbohrvorrichtung gemäss einer ersten Ausführungsform umfassend einen Dentalbohrer mit fünf in Axialrichtung voneinander beabstandete Nuten innerhalb eines Halteabschnitts eines Zylinderschafts und ein als Klemmring ausgebildetes, in eine der Nuten eingesetztes Stoppelement;
- Fig. 2: in perspektivischer Darstellung eine Dentalbohrvorrichtung gemäss einer zweiten Ausführungsform umfassend einen Dentalbohrer und ein Stoppelement, wobei drei in Axialrichtung voneinander beabstandete Verdickungen in einem Halteabschnitt eines Zylinderschafts aufgebildet sind und ein Stoppelement auf eine der Verdickungen aufgesteckt ist;
- Fig. 3a: in perspektivischer Darstellung eine Dentalbohrvorrichtung gemäss einer dritten Ausführungsform umfassend ein Dentalbohrer und ein hülsenförmiges Stoppelement, wobei in einen Haltebereich des Dentalbohrers drei Nuten ausgebildet sind, an welchen das Stoppelement in Axialrichtung fixierbar ist;
- Fig. 3b: in Seitenansicht den Dentalbohrer gemäss Fig. 3a mit geschnitten dargestelltem Stoppelement;
- Fig. 4a: in perspektivischer Darstellung eine erfindungsgemässe Dentalbohrvorrichtung gemäss einer vierten Ausführungsform umfassend einen in ein Verlängerungselement eingesetzten Dentalbohrer und ein am Verlängerungselement gehaltenes Stoppelement;
- Fig. 4b: in perspektivischer Darstellung die Dentalbohrvorrichtung gemäss Fig. 4a, wobei das Stoppelement transparent dargestellt ist;
- Fig. 4c: die Dentalbohrvorrichtung gemäss Fig. 4a und Fig. 4b, wobei der Dentalbohrer in Seitenansicht und das Verlängerungselement und das Stoppelement im Längsschnitt gezeigt sind;
- Fig. 4d: in Seitenansicht die Dentalbohrvorrichtung gemäss Fig. 4a, Fig. 4b und Fig. 4c; und
- Fig. 5: in Schnittdarstellung eine Dentalbohrvorrichtung gemäss einer weiteren Ausbildung der Dentalbohrvorrichtung gemäss Fig. 2, in welcher der Dentalbohrer in einer Bohrlehre geführt ist.

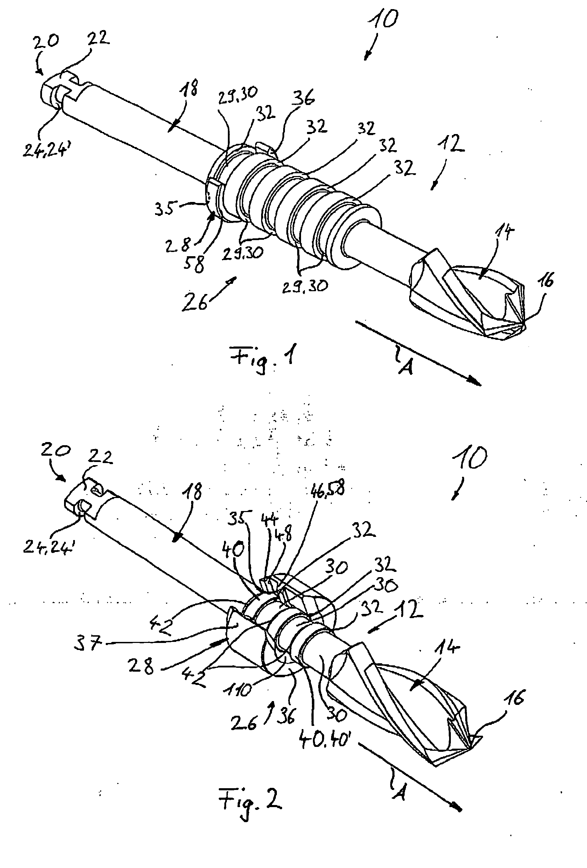

In Fig. 1 ist ein erstes Ausführungsbeispiel einer Dentalbohrvorrichtung 10 gezeigt. Ein Dentalbohrer 12 weist einerseits einen Schneidteil 14 mit einem freiliegenden, stirnseitigen Bohrende 16, und andererseits einen Schaftteil 18 mit einem freiliegenden Aufnahmeendbereich 20 auf. Der Aufnahmeendbereich 20 ist dazu bestimmt, in einer allgemein bekannten Bohrerhaltevorrichtung aufgenommen zu werden und weist eine als Fläche ausgebildete Drehsicherung 22 und eine als teilweise, in Umfangsrichtung verlaufende Nute 24 ausgebildete Axialsicherung 24' auf. Durch die Drehsicherung 22 und die Axialsicherung 24' kann der Dentalbohrer 12 in eine feste Verbindung mit der Bohrerhaltevorrichtung gebracht werden, welche beispielsweise Bestandteil eines Bohrantriebs oder eines Handbohrers ist.

Der Schaftteil 18 weist einen Halteabschnitt 26 für ein Stoppelement 28 auf. Dieser Halteabschnitt 26 ist durch eine kreiszylinderförmige Verdickung des Schaftteils 18 ausgebildet, in welche fünf, als umlaufende Nuten 29 ausgebildete Befestigungsvertiefungen 30 eingeformt sind. In Axialrichtung A des Dentalbohrers 12 weisen alle Nuten 29 dieselbe Breite und in radialer Richtung des Dentalbohrers 12 dieselbe Tiefe auf. In Axialrichtung A ist jede der Befestigungsvertiefungen 30 einerseits, in Richtung des Aufnahmeendbereichs 20 des Schaftteils 18, durch eine rechtwinklig zur Axialrichtung A stehende, kreisringförmige Anschlagsfläche 32 begrenzt. Jede Befestigungsvertiefung 30 ist in Richtung des Bohrendes 16 durch eine weitere, rechtwinklig zur Axialrichtung A stehende, ebenfalls kreisringförmige Begrenzungsfläche begrenzt.

Das Stoppelement 28 der Dentalbohrvorrichtung 10 ist durch einen kreisringförmigen und offenen, somit C-förmigen Klemmring ausgebildet. Das Stoppelement 28 ist dazu bestimmt, in eine der Befestigungsvertiefungen 30, in radialer Richtung klemmend, eingesteckt zu werden. Das Stoppelement 28 bildet ein Eingriffselement 58.

Im folgenden wird das Stoppelement 28 im in eine der Befestigungsvertiefungen 30 eingestecktem Zustand, wie in Fig. 1 gezeigt, beschrieben.

Der Aussendurchmesser des Stoppelements ist grösser gewählt als der Aussendurchmesser des Halteabschnitts 26 und ebenfalls grösser als der Bohrdurchmesser des Schneidteils 14. Die Breite des Klemmrings, in Axialrichtung A gemessen, ist zumindest geringfügig kleiner gewählt als der Abstand der Anschlagfläche 32 zur gegenüberliegenden Begrenzungsfläche der Befestigungsvertiefung 30. Durch diese Ausbildungsform steht das in eine der Befestigungsvertiefungen 30 eingesetzte Stoppelement 28 in radialer Richtung über den Halteabschnitt 26 und über den Schneidteil 14 vor. Eine der Anschlagfläche 32 zugewande, stirnseitige Gegexzanschlagfläche 35 des Stoppelements 28 steht rechtwinklig zur Axialrichtung A und ist dazu bestimmt, mit der Anschlagfläche 32 zusammen zu wirken. In Axialrichtung A der Gegenanschlagfläche 35 gegenüberliegend und dem Bohrende 16 zugewandt weist das Stoppelement 28 eine rechtwinklig zur Axialrichtung A liegende Bohranschlagfläche 36 auf.

Vorzugsweise ist das Stoppelement 28 symmetrisch zu einer rechtwinklig zur Axialrichtung A stehenden Ebene, ausgebildet, sodass die Bohranschlagfläche 36 und die Gegenanschlagfläche 35 gleich ausgebildet sind.

Um die Bohrtiefe eines in den Kieferknochen zu Bohrenden Lochs festzulegen, wird das Stoppelement 28 in die betreffende der Befestigungsvertiefungen 30 eingesetzt. Durch den Abstand der Bohranschlagfläche 36 zum Bohrende 16 ist die maximale Bohrtiefe festgelegt. Falls eine andere maximale Bohrtiefe gewünscht ist, kann das Stoppelement 28 aus der Befestigungsvertiefung 30 entfernt und in eine andere der Befestigungsvertiefungen 30 eingesteckt werden.

Durch die rechtwinklig zur Axialrichtung A liegende Anschlagfläche 32, die mit der ebenfalls rechtwinklig zur Axialrichtung A stehenden Gegenanschlagfläche 35 des Stoppelements 28 zusammenwirkt, ist ein unbeabsichtigtes Verändern der Bohrtiefe mittels eines Formschlusses verhindert. Eine Anpassung der maximalen Bohrtiefe ist dennoch auf einfachste Weise - durch radiales Herausziehen des Stoppelements 28 aus der betreffenden Befestigungsvertiefungen 30 und Einstecken des Stoppelements 28 in eine andere der Befestigungsvertiefungen 30 - möglich.

Im folgenden werden die weiteren Ausführungsbeispiele und Ausführungsformen beschrieben, wobei für gleiche bzw. entsprechende Elemente dieselben Bezugszeichen verwendet werden.

Im in Fig. 2 gezeigten zweiten Ausführungsbeispiel der Dentalbohrvorrichtung 10 ist der Dentalbohrer 12 weitgehend - bis auf die Ausbildung des Halteabschnitts 26 - gleich wie der Dentalbohrer 12 des ersten Ausführungsbeispiels ausgebildet.

Im Halteabschnitt 26 weist der Schaftteil 18 drei umlaufende, manschettenartige Verdickungen 40 auf. Die in Axialrichtung A dem Schneidteil 14 nächstliegende Verdickung 40' ist von dem Schneidteil 14 beabstandet. Jede der Verdickungen 40 weist, dem Bohrende 16 zugewandt, eine rechtwinklig zur Axialrichtung A des Dentalbohrers 12 stehende Anschlagfläche 32 auf. Direkt anschliessend an die Anschlagfläche 32 und in Richtung des Aufnahmeendbereichs 20 weist jede der Verdickungen 40 zunächst einen konstanten Durchmesser auf. Daran anschliessend verjüngen sich jede der Verdickungen 40 auf den Durchmesser des Zylinderschafts 18, Die Verjüngung erfolgt über eine kegelstumpfförmige Klemmfläche 42.

Befestigungsvertiefungen 30 sind zwischen den einzelnen Verdickungen 40 ausgebildet. Eine weitere Befestigungsvertiefung 30 ist zwischen der dem Schneidteil 14 nächstliegenden Verdickung 40' und dem Schneidteil 14 ausgebildet. Jede der Befestigungsvertiefungen 30 ist in Richtung des Aufnahmeendbereichs 20 durch die Anschlagfläche 32 begrenzt.

Das Stoppelement 28 weist eine gerade, kreisrunde Hohlzylinderform mit einer C-förmigen Querschnittsfläche auf, ist im Wesentlichen als offener Klemmring ausgebildet und dazu bestimmt, auf eine der Verdickungen 40, in radialer Richtung, aufgesteckt zu werden. Eine äussere Mantelfläche des Stoppelements 28 liegt auf einem Kreiszylinder mit einem Durchmesser, welcher grösser ist als ein Bohrdurchmesser des Schneidteils 14. Eine der Stirnflächen des Stoppelements 28 bildet eine Bohranschlagsfläche 36 und steht - falls das Stoppelement 28 auf eine der Verdickungen 40 aufgesteckt ist - rechtwinklig zur Axialrichtung A und ist in Richtung des Bohrendes 16 freiliegend.

Die äussere Mantelfläche des Stoppelements 28 bildet eine Führungsoberfläche 37, welche dazu bestimmt ist, mit einer Gegenführungsfläche 106 einer weiter hinten beschriebenen Bohrlehre 100 zusammen zu wirken.

Im Folgenden wird das Stoppelement 28 im auf eine der Verdickungen 40 aufgesteckten Zustand beschrieben.

Das Stoppelement 28 wird durch zwei, voneinander in Axialrichtung A beanstandeten Leisten 44, 46, welche von einer inneren Mantelfläche des C-förmigen Zylinders vorstehen und in Umfangsrichtung verlaufen, jeweils an einer der Verdickungen 40 im Halteabschnitt 26 am Dentalbohrer 12 gehalten. Die dem Aufnahmeendbereich zugewandte Leiste 44 weist hierzu eine mit der Klemmfläche 42 zusammenwirkende Gegenklemmfläche 48 auf. Die andere Leiste 46 bildet ein Eingriffselement 58 und weist eine mit der Anschlagfläche 32 zusammenwirkende Gegenanschlagfläche 35 auf. Die Gegenanschlagfläche 35 steht, wie die Anschlagfläche 32, rechtwinklig zur Axialrichtung A. Die Breite (in Axialrichtung A) der Leisten 44, 46 ist kleiner gewählt als der axialer Abstand der Verdickungen 40 zueinander.

Das auf eine der Verdickungen 40 aufgesteckte Stoppelement 28 liegt mit der inneren Mantelfläche an der in Richtung des Bohrendes 16 direkt nachfolgenden Verdickung 40 an, wodurch das Stoppelement 28 an dieser Verdickung 40 abgestützt ist.

Falls das Stoppelement 28 auf der zum Schneidteil 14 nächstliegenden Verdickung 40' aufgesetzt ist, stützt sich das Stoppelement 28 am Schneidteil 14 ab. Hierzu weist das Schneidteil der Verdickung 40' zugewandt eine Kegelstumpfmantelfläche auf, welche passend zu einer Stützfläche 110 am Stoppelement 28 ausgebildet ist. Die Stützfläche 110 verjungt sich von der Bohranschlagfläche 36 her auf den Durchmesser der inneren Mantelfläche des Stoppelements 28.

Die Handhabung der Dentalbohrvorrichtung 10 gemäss dem zweiten Ausführungsbeispiel ist weitgehend analog zur oben beschriebenen Handhabung des ersten Ausführungsbeispiels der Dentalbohrvorrichtung. Die maximale Bohrtiefe wird wiederum durch den Abstand der Bohranschlagfläche 36 zum Bohrende 16 des Schneidteils 14 bestimmt. Durch radiales Herausziehen des Stoppelements 28 aus dem Halteabschnitt 26 und Aufstecken des Stoppelements 28 auf eine gewünschte der Verdickungen 40 ist die maximale Bohrtiefe veränderbar. Ein unbeabsichtigtes Verändern der maximalen Bohrtiefe ist wiederum durch den Formschluss zwischen der Anschlagsfläche 32 und der mit dieser Anschlagsfläche 32 zusammenwirkenden Gegenanschlagfläche 35 in Axialrichtung A verhindert. Ein fester Sitz in Axialrichtung A des Stoppelements ist durch das Zusammenwirken der Klemmfläche 42 mit der Gegenklemmfläche 48 gewährleistet, wodurch die Gegenanschlagfläche 35 fest an die Anschlagfläche 32 gepresst ist. In radialer Richtung ist das Stoppelement 28 durch die klemmringartige Ausbildung kraftschlüssig am Dentalbohrer 12 fixiert.

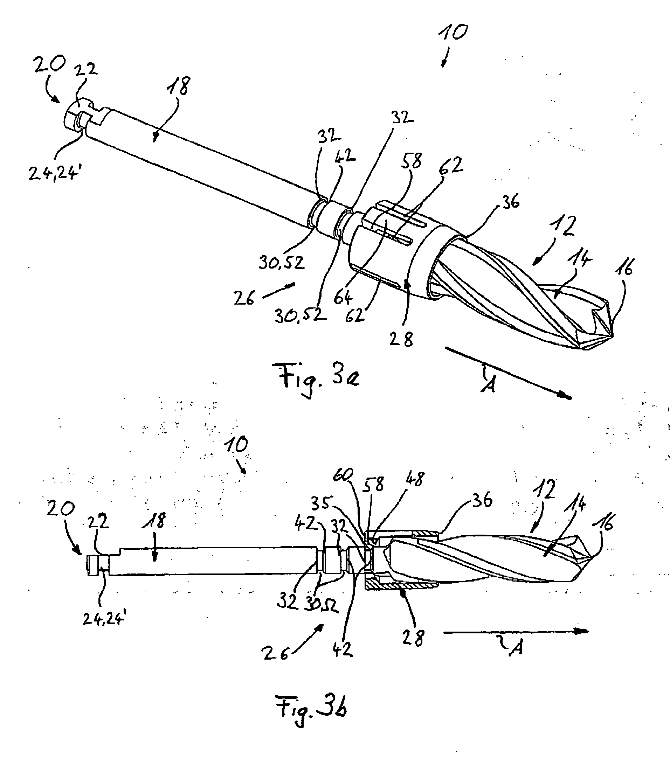

Das dritte Ausführungsbeispiel einer Dentalbohrvorrichtung ist in den Fig. 3a und Fig. 3b gezeigt.

Der Dentalbohrer 12 ist Wiederum weitgehend - bis auf die Ausbildung des Halteabschnitts 26 - gleich wie der Dentalbohrer 12 des ersten Ausführungsbeispiels ausgebildet.

Der einen konstanten Durchmesser aufweisenden Schaftteil 18 weist im Halteabschnitt 26 drei als umlaufende Nuten 52 ausgebildete Befestigungsvertiefungen 30 auf. Der Halteabschnitt 26 grenzt direkt an das Schneidteil 14 an. Jede der Befestigungsvertiefungen 30 ist einerseits durch eine dem Aufnahmeendbereich 20 des Schaftteil 18 zugewandte Anschlagsfläche 32, die rechtwinklig zur Axialrichtung A des Dentalbohrers 12 ausgerichtet ist, und andererseits durch eine rampenartige Klemmfläche 42 begrenzt.

Jede der Befestigungsvertiefungen 30 weist zwischen der Anschlagsfläche 32 und der Klemmfläche 42 eine Kreiszylinderfläche mit konstantem Durchmesser auf. Die Klemmfläche 42 liegt einer eine Kegelstumpfmantolfläche, wobei sie von der Kreiszylinderfläche der Befestigungsvertiefung 30 her rampenartig in Richtung des Bohrendes 16 ansteigt. Der Durchmesser des Schaftteils 18 ist kleiner gewählt als ein Bohrdurchmesser des Schneidteils 14.

Das Stoppelement 28 weist eine hülsenartige Form auf und ist dazu bestimmt, auf den Dentalbohrer 12 vom Aufnahmeendbereich 20 des Schaftteils 18 her aufgesetzt zu werden. Im Folgenden wird wiederum das auf den Dentalbohrer 12 aufgesetzte Stoppelement 28 beschrieben.

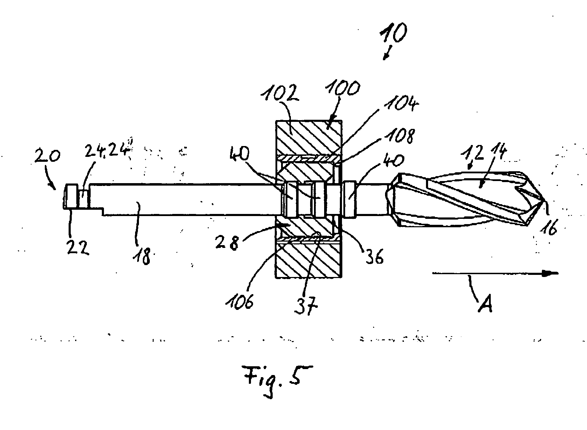

Der Innendurchmesser des Stoppelements 28 ist ausser im Bereich von Eingriffselementen 58, die dazu bestimmt sind, in eine der Befestigungsvertiefungen 30 einzugreifen, im Wesentlichen gleich dem Bohrdurchmesser des Schneidteils 14. Das Stoppelement 28 weist in Axialrichtung A einerseits, in Richtung des Bohrendes 16, eine stirnseitige, rechtwinklig zur Axialrichtung A stehende Bohranschlagfläche 36 und andererseits eine Endfläche 60 auf. Ein axialer, an die Bohranschlagfläche 36 angrenzender Endbereich einer radial innenliegenden Mantelfläche des Stoppelements 28 liegt an den Schneidteil 14 an. Eine auf einer Kreiszylindermantelfläche liegende äussere Mantelfläche des Stoppelements 28 bildet eine Führungsoberfläche 37 und kann mit der Gegenführungsfläche 106 (siehe Fig. 5) der Bohrlehre 100 zusammen wirken. Um gegebenenfalls ein Einführen des Stoppelements 28 in die Bohrlehre 100 zu erleichtern, weist das Stoppelement 28 angrenzend an die Bohranschlagfläche 36 einen Mantelflächenbereich auf, welcher durch eine Kegelstumpffläche ausgebildet ist.

Von der Endfläche 60 her weist das Stoppelement 28 sechs in Axialrichtung A verlaufende, sich in etwa über zwei Drittel der Länge des Stoppelements 28 erstreckende, in Richtung der Endfläche 60 offene Ausnehmungen 62 auf, durch welche drei als Federarme ausgebildete Federelemente 64 am Stoppelement 28 ausgebildet sind. Die Federelemente 64 sind in Umfangsrichtung in regelmässigen Abständen zueinander angeordnet. Jedes Federelement 64 weist an seinem freien Endbereich ein Eingriffselement 58 auf, welches dazu bestimmt sind, in eine der Befestigungsvertiefung 30 einzugreifen. Jedes Eingriffselement 58 weist eine Gegenanschlagsfläche 35 auf, welche in Richtung des Aufnahmeendbereichs 20 freiliegend ist und dazu bestimmt ist, mit der Anschlagfläche 32 zusammenzuwirken, durch welche die Befestigungsvertiefung 30 begrenzt ist. Eine der Gegenanschlagsfläche 55 am Eingriffselement 58 in Axialrichtung A gegenüberliegende Gegenklemmfläche 48 ist dazu bestimmt, mit einer der Klemmflächen 42 der Befestigungsvertiefungen 30 zusammen zu wirken und liegt bei Eingriff des Eingriffselements 58 in eine der Befestigungsvertiefungen 30 an die Klemmfläche 42 der jeweiligen Befestigungsvertiefung 30 an. Durch das Zusammenwirken der Gegenklemmflächen 48 mit der Klemmfläche 42 und durch die Krafteinwirkung der Federelemente 64 werden die Gegenanschlagflächen 35 in Anlage an die Anschlagfläche 32 gedrückt. Dabei stehen die Gegenanschlagflächen 35 ebenfalls rechtwinklig zur Axialrichtung A.

Angewandt wird die Dentalbohrvorrichtung 10 gemäss des dritten Ausführungsbeispiels wie folgt.

Das Stoppelement 28 wird vom Aufnahmeendbereich 20 her, mit der Bohranschlagfläche 36 voraus auf den Dentalbohrer 12 aufgesteckt. Sobald die Gegenklemmflächen 48 der Eingriffelemente 58 in Anlage an den Schaftteil 18 gelangen, werden die Federelemente 64 in radialer Richtung ausgelenkt, wodurch die Gegenanschlagflächen 35 durch elastische Auslenkung in eine nicht rechtwinklige Position zur Axialrichtung A gezwungen werden. Sobald die radial innenliegende Endkanten der Gegenanschlagflächen 35 die Anschlagfläche 32 passiert haben, schnellen die Federelemente 64 in eine parallele Ausrichtung zur Axialrichtung A, wodurch die Eingriffselemente 58 in Eingriff mit den Befestigungsvertiefungen 30 und die Gegenanschlagflächen 35 in die zur Axialrichtung A rechtwinklige Position gebracht werden. Durch die Federkraft der Federelemente 64 und durch Zusammenwirken der Klemmfläche 42 mit den Gegenklemmflächen 48 werden die Gegenanschlagflächen 35 in Anlage an die Anschlagfläche 32 gepresst. Durch den zwischen der Anschlagfläche 32 und den Gegenanschlagflächen 35 wirkenden Formschluss ist das Stoppelement 35 in Richtung des Aufnahmeendbereichs 20 unverschiebbar in einer der Befestigungsvertiefungen 30 gehalten.

Durch Aufbringen einer in Richtung des Bohrendes 16 gerichteten Mindestkraft ist das Stoppelement 28 in Richtung des Bohrendes 16 verschiebbar, so dass die Eingriffselemente 58 des Stoppelements 28 in eine nächste, in Richtung des Bohrendes 16 liegende Befestigungsvertiefung 30 verbringbar sind. Durch die rampenartige Klemmfläche 42 und die Gegenklemmflächen 48 werden die Federelemente 64 bei einer Bewegung des Stoppelements 28 in Richtung des Bohrendes 16 in radialer Richtung ausgelenkt. Der Eingriff der Eingriffselemente 58 in die nächste Befestigungsvertiefung 30 erfolgt wie oben beschrieben. Falls keine weiter Befestigungsvertiefung 30 vorhanden ist, kann das Stoppelement 28 über das Schneidteil 14 abgezogen werden. Die Eingriffselements .58 sind dabei in den Spannuten des Schneidteils 14 geführt.

Das Stoppelement 28 auch des dritten Ausführungsbeispiels ist durch einen Formschluss, falls die Eingriffselemente 58 in Eingriff in eine der Befestigungsvertiefungen 30 sind, in Richtung des freien Endbereichs 20 unverschiebbar am Dentalbohrer 12 gehalten. Dadurch ist eine unbeabsichtigte Vergrösserung des Abstandes der Bohranschlagfläche 36 zum Bohrende 16 verhindert. Hingegen ist eine Verkleinerung des Abstandes der Bohranschlagfläche 36 zum Bohrende 16 durch Verschieben des Stoppelements 28 in Richtung des Bohrendes 16 möglich.

Ein viertes, erfindungsgemässes Ausführungsbeispiel einer bentalbohrvorrichtung ist in den Fig. 4a, Fig. 4b, Fig. 4c und Fig. 4d gezeigt.

Diese Dentalbohrvorrichtung 10 umfasst einen analog zum Dentalbohrer des ersten Ausführungsbeispiels ausgebildeten Dentalbohrer 12, wobei der Dentalbohrer 12 des vierten Ausführungsbeispiels keinen Halteabschnitt aufweist. An den Schneidteil 14 des Dentalbohrers 12 ist ein Bohrerschaft 70 angeformt, welcher wiederum an einem freiliegenden Montageendbereich 76 eine Axialsicherung 24' wie auch eine Drehsicherung 22 - analog zum Dentalbohrer des ersten Ausführungsbeispiels - aufweist. Der Bohrerschaft 70 ist kürzer ausgebildet als der Schaftteil des Dentalbohrers des ersten Ausführungsbeispiels. Der Montageendbereich 76 ist dazu bestimmt, in eine entsprechende Aufnahmevertiefung 72 eines Verlängerungselements 74 aufgenommen zu werden.

Das Verlängerungselement 74 weist einen radial einmal gestuften Schaftteil 18 auf. Der Abschnitt des gestuften Schaftteils 18, der einen grösseren Durchmesser aufweist, bildet einen Halteabschnitt 26. Innerhalb des Halteabschnitts 26 ist der Schaftteil 18 mit der Aufnahmevertiefung 72 versehen. Gegenüberliegend der Aufnahmevertiefung 72 weist der Schaftteil 18 einen Aufnahmeendbereich 20 auf. Der Aufnahmeendbereich 20 ist dazu bestimmt, in einer Bohrhaltevorrichtung aufgenommen zu werden. Dazu weist der Aufnahmeendbereich 20 eine Axialsicherung 24' und eine Drehsicherung 22 auf, welche analog zur Axialsicherung 22 und Drehsicherung 24' des Dentalbohrers 12 ausgebildet sind. Auf den Halteabschnitt 26 ist ein hülsenförmiges Stoppelement 28 aufsteckbar.

Im folgenden ist die Dentalbohrvorrichtung 10 in einem Zustand beschrieben, in welchem der Montageendbereich 76 des Dentalbohrers 12 in der Aufnahmevertiefung 72 durch ein, in die Axialsicherung 24' eingreifendes Federelement, vorzugsweise ein Federarm 77 gehalten ist. Weiter ist das Stoppelement 28 vom Bohrende 16 her auf den Halteabschnitt 26 aufgeschoben.

Der Halteabschnitt 26 weist im Wesentlichen eine radial äussere, kreiszylindrische Mantelfläche auf, welche als Führungsfläche für ein hülsenartiges Stoppelement 28 dient. Der Aussendurchmesser des Halteabschnitts 26 ist leicht grösser als der Bohrdurchmesser des Dentalbohrers 12 gewählt. Im Halteabschnitt 26 ist eine kanalartige, durch Nuten ausgebildete Struktur eingelassen. Eine Führungsnute 78 verläuft in Axialrichtung A des Dentalbohrers 12 und ist in Richtung des Bohrendes 16 offen. Von der Führungsnut 78 führen in Umfangsrichtung, alle in dieselbe Richtung weglaufend, drei als Umfangsnuten 80 ausgebildete Befestigungsvertiefungen 30 weg. Die Umfangsnuten 80 weisen alle dieselbe Länge und in Umfangsrichtung an ihren, der Führungsnut 78 entfernten Endbereichen eine Rastvertiefung 82 auf. Querschnitte der Führungsnute 78 (in Umfangsrichtung) und jene der Umfangsnuten 80 (in Axialrichtung) sind im wesentlichen gleich und weisen angrenzend an die Mantelfläche parallel zur radialen Richtung verlaufende seitenwände 83 auf. Ein Nutenboden der Führungsnut 78 wie auch der Umfangsnuten 80 ist zylindrisch ausgebildet. Die Rastvertiefungen 82 sind in radialer Richtung tiefer ausgebildet als der restliche Bereich der Befestigungsvertiefungen 30, wobei die Seitenwände 83 in radialer Richtung verlängert sind und der Nutenboden im Bereich der Befestigungsvertiefung 30 dadurch abgesenkt ist.

Jede Befestigungsvertiefung 30 weist eine Anschlagfläche 32 auf, die zumindest nahezu rechtwinklig zur Axialrichtung A steht und durch einen Abschnitt der Seitenwand 83 ausgebildet ist, der die Befestigungsvertiefung in Richtung des Aufnahmeendbereichs 20 begrenzt.

Das auf den Haltebereich 26 aufsetzbare Stoppelement 28 ist hülsenartig ausgeführt. In Axialrichtung A weist das Stoppelement 28 einerseits, in Richtung des Bohrendes 16 eine rechtwinklig zur Axialrichtung A stehende, stirnseitige Bohranschlagfläche 36 und andererseits eine stirnseitige Endfläche 60 auf. Eine auf einer Kreiszylindermantelfläche liegende äussere Mantelfläche des Stoppelements 28 bildet eine Führungsoberfläche 37.

Die Führungsoberfläche 37 wirkt bei Verwendung einer, in Zusammenhang mit Fig. 5 näher beschriebenen Bohrlehre 100 mit einer Gegenführungsfläche 106 der Bohrlehre 100 zusammen.

Ein in Umfangsrichtung angeordnetes, als Federfinger ausgebildete Federelement 84 ist durch eine schlitzartige Ausnehmung 86 am Stoppelement 28 ausgebildet. Die Ausnehmung 86 verläuft von der Endfläche 60 her zunächst in Axialrichtung A und dann in Umgangsrichtung.

Am freien Endbereich des Federelements 84 ist ein radial nach innen vorstehendes, stiftartiges Eingriffselement 58 befestigt, welches dazu bestimmt ist, in die Nuten 78, 80 einzugreifen. Das Eingriffselement 58 weist direkt anschliessend an eine radial innenliegende Fläche des Federelements 84 einen kreiszylindrischen Abschnitt und daran in radialer Richtung anschliessend einen halbkugelförmigen Abschnitt auf, der dazu bestimmt ist, mit einer der Rastvertiefungen 82 zusammen zu wirken. Der kreiszylindrische Abschnitt bildet eine Gegenanschlagsfläche 35, welche dazu bestimmt ist, mit einer der Anschlagsflächen 32 zusammen zu wirken. Der Durchmesser des halbkugelförmigen und des zylindrischen Abschnitts ist derart gewählt, dass das Eingriffselement 58 in der Befestigungsvertiefung 30 in Axialrichtung A wenig Spiel hat.

Die Dentalbohrvorrichtung 10 gemäss dem vierten Ausführungsbeispiels wird wie folgt angewendet.

Der Dentalbohrer 12 wird mit dem Montagebereich 76 voraus in die Aufnahmeöffnung 72 des Verlängerungselements 74 eingesetzt, wodurch der Federarm 77 in Eingriff in die Axialsicherung 24' des Dentalbohrers 12 gelangt.

Auf den Dentalbohrer 12 wird vom Bohrende 16 her und mit der Endfläche 60 voraus das Stoppelement 28 aufgesetzt und in Axialrichtung A in Richtung des Halteabschnitts 26 bewegt. Beim Aufsetzten des Stoppelements auf den Halteabschnitt 26 ist darauf zu achten, dass das Eingriffselement 58 mit der Führungsnute 78 fluchtet. Bei Weiterbewegung des Stoppelements 28 ist das Eingriffselement 58 in der Führungsnute 78 geführt.

Durch das Aufschieben des Stoppelements 28 ist eine radiale Auslenkung des Federarms 77 verunmöglicht, wodurch der Dentalbohrer 12 in der Aufnahmevertiefung 72 des Verlängerungselements 74 gesichert wird.

Falls das Eingriffselement 58 durch Verschiebung des Stoppelements 28 in Axialrichtung A mit der gewünschten Befestigungsvertiefungen 30 in Ausrichtung gelangt, kann das Stoppelement in Umfangsrichtung gedreht werden, wodurch das Eingriffelement 58 in die Rastvertiefung 82 der Befestigungsvertiefung 30 einrastet.

Bei Eingriff des Eingriffselement 58 in eine der Befestigungsvertiefungen 30 ist das Stoppelement 28 durch einen Formschluss zwischen der Anschlagfläche 32 und der Gegenanschlagfläche 35 in Axialrichtung A, insbesondere in Richtung des Aufnahmeendbereichs 20 des Zylinderschafts 18 gesichert. In Umfangsrichtung ist das Stoppelement 28 bei Eingriff des Eingriffelements 58 in eine der Rastvertiefungen 83 durch einen Kraftschluss verdrehsicher gehalten.

Die gewünschte maximale Bohrtiefe der Dentalbohrvorrichtung 10 gemäss dem vierten Ausführungsbeispiel ist wiederum durch den Abstand der Bohranschlagfläche 36 vom Bohrende 16-festgelegt.

Die maximalen Bohrtiefe ist wiederum auf einfachste Weise veränderbar, Hierzu wird zunächst das Stoppelement 28 in Umfangsrichtung und in Richtung der Führungsnute 78 gedreht, sodass das Eingriffselement 58 durch Überwinden des Kraftschlusses aus der Rastvertiefung 82, in welche das Eingriffselement 58 eingreift, herausbewegt wird. Anschliessende wird das Stoppelement 28 in Axialrichtung A bewegt, das Eingriffselement 58 mit einer anderen Befestigungsvertiefung 30 ausgerichtet und durch eine Drehung des Stoppelements 28 gelangt das Eingriffselement 58 in Eingriff in die Befestigungsvertiefung 30.

Es ist ebenfalls denkbar, dass das Stoppelement, 28 am Verlängerungselement 74 im Halteabschnitt 26. vormontiert ist. In diesem Fall weist der Federarm 77 gegenüber dem Stoppelement 28 eine so grosse Bewegungsfreiheit auf, dass beim Einsetzten des Montagebereichs 76 des Dentalbohrers 12 in die Aufnahmeöffnung 72, der Federarm 77 zum Einrasten in die Axialsicherung 24' auslenkbar ist.

Bei den oben beschriebenen Ausführungsbeispielen wird die gewünschte Bohrtiefe durch den Abstand der Bohranschlagfläche 36 zum Bohrende 16 festgelegt. Beim erreichen der gewünschten Bohrtiefe beim Bohren eines Lochs in einen Kieferknochen, gelangt die Bohranschlagfläche in Anlage an den Kieferknochen beziehungsweise an das den Kieferknochen umgebende Zahnfleisch.

In Fig. 5 ist eine weitere Ausbildung der Dentalbohrvorrichtung gemäss dem zweiten Ausführungsbeispiel gezeigt. Diese Ausbildung weist eine Bohrlehre 100 auf, in welcher der Dentalbohrer 12 mit dem daran angeordneten Stoppelement 28 geführt ist.

Die Bohrlehre 100 ist dazu bestimmt, ortsfest zu einer Mundhöhle eines Patienten befestigt zu werden. Vorzugsweise wird die Bohrlehre 100 aufgrund eines Abdrucks des Oberkiefers beziehungsweise des Unterkiefers hergestellt. Die Bohrlehre 100 weist eine Bohrlehrenplatte 102 mit einer darin fest eingelassenen Bohrlehrenhülse 104 auf. Eine radial innenliegende Oberfläche der Bohrlehrenhülse 104 bildet eine Gegenführungsfläche 106 zur Führungsoberfläche 37 des Stoppelements 28. An einem axialen Endbereich weist die Bohrlehrenhülse 104 eine radial nach innen vorstehende, ringförmige Schulter 108 auf. Die Schulter 108 bildet einen Gegenanschlag für die Bohranschlagfläche 36 des Stoppelements 28.

Angewandt wird die in Fig. 5 gezeigte Dentalbohrvorrichtung 10 wie folgt.

Die Bohrlehre 100 wird für eine Behandlung eines Patienten in dessen Mundhöhle bzw. in unmittelbarer Nähe zu ihr und ortsfest zur Mundhöhle angeordnet. Die Schulter 108 der Bohrlehre 100 ist dem zu bohrenden Loch, das heisst dem Kieferknochen zugewandt. Die gewünschte Bohrtiefe wird mittels des Stoppelements 28 am Dentalbohrer 12 durch Einstecken des Stoppelements auf eine der Verdickungen 40 festgelegt. Die gewünschte, maximale Bohrtiefe ist in diesem Ausführungsbeispiel durch den Abstand der Bohranschlagfläche 36 zur Schulter 108 bestimmt. Durch Einführen des Stoppelements in die durch die Bohrhülse 104 gebildete Ausnehmung in der Bohrlehre und durch das Zusammenwirken der Führungsoberfläche 37 des Stoppelements 28 mit der Gegenführungsfläche 106 ist die Lage und Richtung des Bohrlochs definiert.

Folglich ist mittels der Dentalbohrvorrichtung 10 gemäss Fig. 5 ein Bohren eines Lochs an gewünschter Lage, in gewünschter Richtung und in gewünschter Tiefe auf einfachste Weise möglich. Eine Beeinträchtigung der Sicht während des Bohrvorgangs, verursacht durch Material aus dem Bohrloch, hat keinen Einfluss auf die Präzision des zu bohrenden Lochs.

Es ist ebenfalls denkbar, auf die Schulter 108 an der Bohrlehre 100 zu verzichten. Folglich ist die maximale Bohrtiefe wiederum durch den Abstand der Bohranschlagfläche 36 vom Bohrende 16 gegeben. Beim Erreichen der maximalen Bohrtiefe liegt die Bohranschlagfläche 36 an den Kieferknochen beziehungsweise an das Zahnfleisch an.

Selbstverständlich ist die Anwendung der Bohrlehre 100 nicht nur auf Dentalbohrer gemäss dem zweiten Ausführungsbeispiels beschränkt. Ein Dentalbohrer gemäss dem dritten Ausführungsbeispiel oder ein Dentalbohrer in Kombination mit einem Verlängerungselement gemäss dem vierten Ausführungsbeispiel kann ebenso mit der Bohrlehre verwendet werden.

Falls auf die Bohrlehre beim Bohren eines Lochs verzichtet wird, kann die radiale Führungsoberfläche 37 des dritten und vierten Ausführungsbeispiels dennoch eine Führung beim Bohren erleichtern, indem die Führungsoberfläche 37 seitlich an einem Element angelehnt wird.

Die Anzahl der Befestigungsvertiefungen in allen Ausführungsbeispielen kann selbstverständlich nahezu beliebig variiert werden und ist nicht auf die in den Ausführungsbeispielen angegebenen Anzahlen beschränkt.

Ebenso sind die ersten drei Ausführungsbeispiele, welche kein Verlängerungselement aufweisen, mit einem Verlängerungselement - analog zum vierten Ausführungsbeispiel - realisierbar. Der Halteabschnitt ist dabei nicht am Dentalbohrer sondern am Verlängerungselement angeordnet. Folglich ist das Stoppelement nicht am Dentalbohrer sondern am Verlängerungselement gehalten. Die notwendigen Anpassungen sind für den Fachmann sofort ersichtlich.

Das vierte Ausführungsbeispiel kann ebenso ohne Verlängerungselement ausgebildet sein. Der Halteabschnitt ist dabei am Schaftteil des Dentalbohrers - analog zu den Dentalbohrern des ersten bis dritten Ausführungsbeispiels - angeordnet. Das Stoppelement ist folglich am Dentalbohrer gehalten. Die notwendigen Anpassungen sind für den Fachmann wiederum klar ersichtlich.

## Patentansprüche

1. Dentalbohrvorrichtung mit einem eine Axialrichtung (A) definierenden Bohrer (12), einem Verlängerungselement (74) und einem an diesem angeordneten Stoppelement (28), wobei der Bohrer (12) einerseits einen Schneidteil (14) mit einem stirnseitigen Bohrende (16) aufweist und dazu bestimmt ist, andererseits am Verlängerungselement (74) gehalten zu sein, das Verlängerungselement (74) einen Aufnahmeendbereich (20), welcher dazu bestimmt ist, in einer Bohrerhaltevorrichtung aufgenommen zu werden, sowie einen Halteabschnitt (26) für das Stoppelement (28) und das Stoppelement (28) eine in Richtung des Bohrendes (16) freiliegende Bohranschlagfläche (36) aufweist, welche in einem gewünschten Abstand zum Bohrende (16) am Verlängerungselement (74) gehalten ist, wobei hierzu am Verlängerungselement (74) innerhalb des Halteabschnitts (26) mehrere in Axialrichtung (A) des Verlängerungselements (74) voneinander beabstandete Befestigungsvertiefungen (30) angeordnet sind und das Stoppelement (28) ein Eingriffselement (58) aufweist, das dazu bestimmt ist, in eine der Befestigungsvertiefungen (30) einzugreifen, wobei die Befestigungsvertiefungen (30) auf ihrer dem Aufnahmeendbereich (20) zugewandten Seite durch eine zumindest annähernd rechtwinklig zur Axialrichtung (A) verlaufende Anschlagfläche (32) begrenzt sind, und das Eingriffselement (58) eine Gegenanschlagfläche (35) aufweist, welche formschlüssig mit der betreffenden Anschlagfläche (32) zusammen wirkt.

2. Dentalbohrvorrichtung gemäss Anspruch 1, **dadurch gekennzeichnet, dass** das Stoppelement (28) eine radial aussenliegende, kreiszylindrische Führungsoberfläche (37) aufweist.

3. Dentalbohrvorrichtung gemäss Anspruch 2, **gekennzeichnet durch** eine Bohrlehre (100), die ortsfest zu einer Mundhöhle eines Patienten fixierbar ist und eine Ausnehmung mit einer kreiszylindrischen Gegenführungsfläche (106) aufweist, die dazu bestimmt ist, mit der Führungsoberfläche (37) des Stoppelements (28) zusammen zu wirken.

4. Dentalbohrvorrichtung gemäss Anspruch 3, **dadurch gekennzeichnet, dass** die Bohrlehre (100) eine in Bezug auf die Gegenführungsfläche (106) radial nach innen vorstehende Schulter (108) aufweist, die dazu bestimmt ist, mit der Bohranschlagsfläche (36) des Stoppelements (28) zusammen zu wirken.

5. Dentalbothrvorrichtung gemäss einem der Ansprüche 1 bis 4, **dadurch gekennzeichnet, dass** jeder der Anschlagflächen (32) eine kegelstumpfförmige Klemmfläche (42) zugeordnet ist, welche dazu bestimmt ist, mit dem Stoppelement (28) zusammen zu wirken und dadurch die Gegenanschlagfläche (35) in Anlage an der Anschlagfläche (32) zu halten.

6. Dentalbohrvorrichtung gemäss Anspruch 5, **dadurch gekennzeichnet, dass** die Klemmfläche (42) mit dem Eingriffselement (58) zusammen wirkt.

7. Dentalbohrvorrichtung gemäss einem der Ansprüche 1 bis 4, **dadurch gekennzeichnet, dass** jede Befestigungsvertiefung (30) eine Rastvertiefung (82) aufweist und das Eingriffselement (58) kraftschlüssig in die betreffende Rastvertiefung (82) eingreift, um das Stoppelement (28) in Umfangsrichtung zu fixieren.

8. Dentalbohrvorrichtung gemäss einem der Ansprüche 1 bis 7, **dadurch gekennzeichnet, dass** das Stoppelement (28) hülsenartig ausgebildet ist und das Eingriffselement (58) vorzugsweise an einem als Federfinger ausgebildeten Federelement (64, 84) angeordnet ist.

9. Dentalbohrvorrichtung gemäss einem der Ansprüche 1 bis 8, **dadurch gekennzeichnet, dass** das Stoppelement (28) eine, vorzugsweise an die Bohranschlagfläche (36) angrenzende, radial innenliegende Mantelfläche aufweist, welche am Schneidteil (14) anliegt.

## Claims

1. Dental drill device with a drill (12) defining an axial direction (A), with an extension element (74), and with a stop element (28) arranged on the latter, the drill (12) having, at one end, a cutting part (14) with a front drill end (16) and being intended, at the other end, to be held on the extension element (74), the extension element (74) having a receiving end area (20) which is intended to be received in a drill holder device, and a holder portion (26) for the stop element (28), and the stop element (28) having a drill abutment surface (36) which is exposed in the direction of the drill end (16) and which is held on the extension element (74) at a desired distance from the drill end (16), for which purpose several fastening recesses (30) spaced apart from one another in the axial direction (A) of the extension element (74) are arranged thereon within the holder portion (26), and the stop element (28) has an engagement element (58) which is intended to engage in one of the fastening recesses (30), said fastening recesses (30) being limited, on their side directed toward the receiving end area (20), by an abutment surface (32) extending at least approximately at right angles to the axial direction (A), and the engagement element (58) having a counter-abutment surface (35) which cooperates in a form fit with the relevant abutment surface (32).

2. Dental drill device according to Claim 1, **characterized in that** the stop element (28) has a radially outward, circular-cylindrical guide surface (37).

3. Dental drill device according to Claim 2, **characterized by** a drill jig (100) which can be fixed in a stationary position relative to the oral cavity of a patient and has a recess with a circular-cylindrical mating guide surface (106) which is intended to cooperate with the guide surface (37) of the stop element (28).

4. Dental drill device according to Claim 3, **characterized in that** the drill jig (100) has, in relation to the mating guide surface (106), a radially inwardly projecting shoulder (108) which is intended to cooperate with the drill abutment surface (36) of the stop element (28).

5. Dental drill device according to one of Claims 1 to 4, **characterized in that** each of the abutment surfaces (32) is assigned a truncated-cone-shaped clamp surface (42) which is intended to cooperate with the stop element (28) and in this way to keep the counter-abutment surface (35) bearing on the abutment surface (32).

6. Dental drill device according to Claim 5, **characterized in that** the clamp surface (42) cooperates with the engagement element (58).

7. Dental drill device according to one of Claims 1 to 4, **characterized in that** each fastening recess (30) has a locking recess (82), and the engagement element (58) engages with a force fit in the relevant locking recess (82) in order to fix the stop element (28) in the circumferential direction.

8. Dental drill device according to one of Claims 1 to 7, **characterized in that** the stop element (28) has a sleeve-shaped design, and the engagement element (58) is preferably arranged on a spring element (64, 84) designed as a resilient finger.

9. Dental drill device according to one of Claims 1 to 8, **characterized in that** the stop element (28) has a radially inward jacket surface which preferably adjoins the drill abutment surface (36) and which bears on the cutting part (14).

## Revendications

1. Dispositif à fraiser dentaire comprenant une fraise (12) définissant une direction axiale (A), un élément de prolongement (74) et un élément d'arrêt (28) disposé sur cet élément, la fraise (12) présentant d'une part une partie de coupe (14) avec une extrémité de fraisage (16) côté avant et est destinée d'autre part à être maintenue sur l'élément de prolongement (74), l'élément de prolongement (74) présentant une zone d'extrémité de réception (20) qui est destinée à être réceptionnée dans un dispositif de retenue de fraise, et une partie de retenue (26) pour l'élément d'arrêt (28) et l'élément d'arrêt (28) présentant une surface de butée de fraisage (36) mise à nu en direction de l'extrémité de fraisage (16), laquelle est maintenue à une distance souhaitée de l'extrémité de fraisage (16) sur l'élément de prolongement (74), plusieurs cavités de fixation (30) espacées les unes des autres dans la direction axiale (A) de l'élément de prolongement (74) étant disposées à cet effet sur l'élément de prolongement (74) à l'intérieur de la partie de retenue (26) et l'élément d'arrêt (28) présentant un élément d'engagement (58) qui est destiné à s'engager dans l'une des cavités de fixation (30), les cavités de fixation (30) étant limitées sur leur côté tourné vers la zone d'extrémité de logement (20) par une surface de butée (32) agencée au moins approximativement à angle droit par rapport à la direction axiale (A), et l'élément d'engagement (58) présentant une surface de contrebutée (35), qui coopère par complémentarité de forme avec la surface de butée (32) concernée.

2. Dispositif de fraisage dentaire selon la revendication 1, **caractérisé en ce que** l'élément d'arrêt (28) présente une surface de guidage (37) cylindrique circulaire et disposée radialement à l'extérieur.

3. Dispositif de fraisage dentaire selon la revendication 2, **caractérisé par** un calibre de fraisage (100), qui peut être fixé de façon fixe à une cavité buccale d'un patient et présente un évidement avec une surface de contre-guidage (106) cylindrique circulaire, qui est destinée à coopérer avec la surface de guidage (37) de l'élément d'arrêt (28).

4. Dispositif de fraisage dentaire selon la revendication 3, **caractérisé en ce que** le calibre de fraisage (100) présente un également (108) dépassant par rapport à la surface de contre-guidage (106) radialement vers l'intérieur, qui est destiné à coopérer avec la surface de butée de fraisage (36) de l'élément d'arrêt (28).

5. Dispositif de fraisage dentaire selon l'une quelconque des revendications 1 à 4, **caractérisé en ce qu'**à chacune des surfaces de butée (32) est attribuée une surface de serrage (42) en forme de cône tronqué, qui est destinée à coopérer avec l'élément d'arrêt (28) et de ce fait, à maintenir la surface de contrebutée (35) en appui sur la surface de butée (32).

6. Dispositif de fraisage dentaire selon la revendication 5, **caractérisé en ce que** la surface de serrage (42) coopère avec l'élément d'engagement (58).

7. Dispositif de fraisage dentaire selon l'une quelconque des revendications 1 à 4, **caractérisé en ce que** chaque cavité de fixation (30) présente une cavité d'encliquetage (82) et l'élément d'engagement (58) s'engage par force dans la cavité d'encliquetage (82) concernée, afin de fixer l'élément d'arrêt (28) dans le sens périphérique.

8. Dispositif de fraisage dentaire selon l'une quelconque des revendications 1 à 7, **caractérisé en ce que** l'élément d'arrêt (28) est conçu en forme de douille et l'élément d'engagement (58) est disposé de préférence sur un élément de ressort (64, 84) réalisé sous forme de doigt de ressort.

9. Dispositif de fraisage dentaire selon l'une quelconque des revendications 1 à 8, **caractérisé en ce que** l'élément d'arrêt (28) présente une surface d'enveloppe, de préférence contiguë à la surface de butée de fraisage (36), radialement intérieure, qui s'appuie sur la partie de coupe (14).
